Europäisches Patentamt

European Patent Office

Office européen des brevets

(11) Veröffentlichungsnummer: **0 271 741 B1**

## (12) EUROPÄISCHE PATENTSCHRIFT

(45) Veröffentlichungstag der Patentschrift: **28.10.92**

(21) Anmeldenummer: **87117194.8**

(22) Anmeldetag: **21.11.87**

(51) Int. Cl.⁵: **A61B 17/08**

(54) **Vorrichtung zum Verschliessen einer Wunde.**

(30) Priorität: **16.12.86 DE 3642892**
**28.02.87 DE 3706599**

(43) Veröffentlichungstag der Anmeldung:
**22.06.88 Patentblatt 88/25**

(45) Bekanntmachung des Hinweises auf die
Patenterteilung:
**28.10.92 Patentblatt 92/44**

(84) Benannte Vertragsstaaten:
**AT BE CH ES FR GB GR IT LI LU NL SE**

(56) Entgegenhaltungen:
**FR-A- 2 210 107**
**GB-A- 1 534 374**
**US-A- 2 012 755**
**US-A- 3 516 409**

**Arch. Surg. vol.121, Feb 1986, Seiten 147-152;
W Teichmann et al: "Scheduled Reoperations (Etappenlavage) for Diffuse Peritonitis"**

(73) Patentinhaber: **Opti Patent-, Forschungs- und
Fabrikations-AG**

**CH-8750 Riedern-Allmeind(CH)**

(72) Erfinder: **Kaessmann, Hans-Jürgen,
Prof.Dr.med.habil.
Meisenstrasse 6
W-2980 Norden-Norddeich(DE)**

(74) Vertreter: **Andrejewski, Walter, Dr. et al
Patentanwälte Andrejewski, Honke & Partner
Postfach 10 02 54 Theaterplatz 3
W-4300 Essen 1(DE)**

**Beschreibung**

Die Erfindung bezieht sich gattungsgemäß auf eine Vorrichtung zum Verschließen einer Wunde, besondere einer hauptsächlich in gerader Richtung verlaufenden Operationswunde, - mit aus zwei textilen Trabändern und daran angeordneten Verschlußgliederreihen sowie einem Reißverschlußschieber aufgebautem Reißverschluß, wobei der Reißverschluß eine Einrichtung zur Klebverbindung mit der Haut eines Patienten oder eines Tieres längs des Wundrandes aufweisen. Der Ausdruck Verschließen bedeutet im Rahmen der Erfindung, daß die Vorrichtung nicht appliziert wird, wenn die Wunde bereits mit Hilfsmitteln geschlossen ist, sondern dazu dient, die zunächst klaffenden Wundränder einer Operationswunde zusammenziehen und gegeneinanderzustoßen und dadurch die Wunde zu schließen Auf die üblichen wundverschließenden Maßnahmen, wie Nähen oder Klammern, soll verzichtet werden.

Bei den bekannten Vorrichtung der angegebenen Zweckbestimmung (US 35 16 409, DE 34 44 782) ist der Reißverschluß ein besonderer, für die Anwendung im Textilbereich üblicherweise nicht verwendbarer Reißverschluß mit besonders eingerichteten Tragbändern, die eine Kleberauflage aufweisen Sie haben sich in die medizinische Praxis nicht eingeführt, weil ihre Sonderanfertigung zu aufwendig ist, die Wundränder mit den Tragbändern und/oder den Verschlußgliedern verwachsen, zumal nicht sichergestellt ist, daß das Wundsekret ablaufen kann. Häufig löst sich die Klebverbindung mit der Haut vorzeitig. Arbeitet man mit einem besonderen, an eines der Tragbänder angeschlossenen Abdeckstreifen, der die Wunde überdeckt, so kann nicht ausgeschlossen werden, daß dieser in die Wunde gelangt oder sich zusammenschiebt. Schiebt er sich zusammen, so liegen in diesem Bereich die Wundränder nicht mehr in einer Ebene nebeneinander, sie wachsen unter Bildung von häßlichen Narben zusammen. Um die Verbindung mit der Haut zu verbessert, kennt man in der Praxis auch für den Wundverschluß eingerichtete Reißverschlüsse, bei denen die Tragbänder jeweils in einer Reihe angeordnete nadelartige Stifte aufweisen, die bei geöffnetem Reißverschluß längs des Wundrandes in die Haut des Patienten oder eines Tieres eingedrückt werden, was umständlich ist und zusätzliche Wunden sowie deutliche Narben verursacht. Zum Schließen der Wunde wird im Rahmen der bekannten Maßnahmen der Wundrand zunächst von Hand zusammengedrückt und danach wird der Reißverschluß geschlossen. All dieses ist der Grund, weshalb die bekannten Maßnahmen bisher lediglich im Abdominalraum eingesetzt wurden (vgl. "Neue Züricher Zeitung" vom 22. August 1984, "Hamburger Abendblatt" von Pfingsten 1984), und zwar lediglich zum temporären Verschließen einer Wunde zum Zwecke der häufigen Öffnung, wie sie z.B. bei Bauchfellentzündungen bei Verfall der Bauchspeicheldrüse erforderlich ist. Dies zeigt auch der Artikel in Arch. Surg. vol. 121, Feb. 1986, S. 147-152 : "Scheduled Reoperations (Etappenlavage) for Diffuse Peritonitis", der die Verwendung eines gewöhnlichen Reißverschlusses erwähnt. Die bekannten Maßnahmen sind zum Verschließen einer Wunde und zum Verschluß einer Wunde beim Heilungsprozeß und damit als Ersatz oder Verbesserung für Wundverschlüsse durch klassisches Nähen oder Klammern nicht geeignet.

Der Erfindung liegt die Aufgabe zugrunde, eine gattungsgemäße Vorrichtung so weiter auszubilden, daß sie zum Verschließen der Wunde und beim Heilungsprozeß als ein eine Verbesserung darstellender Ersatz für den klassischen Wundverschluß durch Nähen oder Klammern eingesetzt werden kann, und zwar bei einfachem, anwendungsfreundlichem Aufbau, der von einfachen Ausgangsbauteilen ausgeht und darüber hinaus sterilisierfreundlich ist. Es versteht sich, daß die Vorrichtung insgesamt beim Hersteller oder vor dem Einsatz mit üblichen Hilfsmitteln sterilisiert wird und entsprechend sterilisationsfest sein muß.

Die Lösung dieser Aufgabe ist gekennzeichnet durch die Kombination der folgenden Merkmale:

1) Der Reißverschluß ist in bezug auf die Verschlußgliederreihen und die Tragbänder ein für textile Kleidungsstücke üblicher, flexibler Reißverschluß mit dehnungsarmen Tragbändern und im gekuppelten Zustand stauchfesten Verschlußgliederreihen aus Polyester und Polyamid,

2) auf der der Wunde zugekehrten Unterseite sind mit Abstand von den Verschlußgliederreihen auf den Tragbändern wundfreiraumbildende Distanzstreifen angeordnet,

3) die Einrichtung zur Klebverbindung besteht aus mit den Tragbändern verbundenen, mit einem Überstand über die Tragbänder überstehenden Klebestreifen mit hautseitiger hypoallergener Klebeschicht,

wobei die Tragbänder von den Klebestreifen zumindest über einen Teil ihrer Breite unterlegt sind und die Klebestreifen einschließlich der Überstände eine Breite aufweisen, die zur Aufnahme der Querzugkräfte bei durch den Reißverschluß geschlossener Wunde mit gegeneinandergedrückten Wundrändern geeignet ist. - Für textile Kleidungsstücke übliche, flexible Reißverschlüsse mit dehnungsarmen Tragbändern und stauchfesten Verschlußgliederreihen sind für die verschiedensten Zwecke eingesetzt worden. In der Fachwelt war man bisher jedoch der Meinung, daß zum Verschließen einer Wunde und als Wundverschluß bei der Heilung solche Reißverschlüsse nicht geeignet sind (vgl.

"Surgical use of zippers worries manufacturer" in China Post vom 08. November 1986).

Es versteht sich, daß die erfindungsgemäße Vorrichtung so appliziert wird, daß eine Operationswunde im geschlossenen Zustand des Reißverschlusses möglichst genau unter der Reißverschlußmitte verläuft. Durch die Kombination der beschriebenen Merkmale erreicht man, daß auf die Wundränder, die durch Schließen des Reißverschlusses gleichsam gegeneinander gestoßen werden, von den Klebestreifen her Kräfte in sehr gleichmäßiger Verteilung, ohne Bereiche singulärer Beanspruchung, ausgeübt werden, die die Wundränder gegeneinander halten, wobei die gekuppelte Verschlußgliederreihe gleichsam schienend wirkt, weil sie stauchfest ausgebildet ist. Dabei liegen die Wundränder in dem Freiraum ausreichend frei, was den Abfluß von Sekret zuläßt, Luft an die Wunde heranläßt und die Heilung unterstützt und fördert. Wegen der Anordnung des Freiraumes ist fernerhin ausreichend sichergestellt, daß die Verschlußglieder der Verschlußgliederreihen nicht einwachsen. Im Rahmen der Erfindung liegt es, die Verschlußgliederreihen auf der Unterseite, gegebenenfalls einschließlich der Tragbänder, mit einer wundfreundlichen Schicht aus Silikonmasse zu beschichten. Endlich wird durch den Freiraum sichergestellt, daß der Schieber beim Verschließen der Wunde bewegt werden kann, ohne daß er mit den Wundrändern in störende Berührung kommt oder die Gefahr besteht, daß Wundränder beim Kuppelvorgang der Verschlußgliederreihen in diese eingezogen werden. Im Ergebnis wachsen die Wundränder wesentlich besser und störungsfreier zusammen als überweise bei Nähnähten oder Klammernähten. Der Heilungsprozeß und die Optik erfahren keine Störungen durch Singularitäten, die entstehen, wenn mit über die Länge der Wunde verteilten Nähnahtstichen oder Klammeren gearbeitet wird. All die beschriebenen Vorteile gelten insgesondere dann, wenn die Operationsnaht vor der Applikation der erfindungsgemäßen Vorrichtung, z.B. mit Einzinkerhäkchen, in Längsrichtung ein wenig unter Zugspannung gesetzt wird, wie es weiter unten erläutert wird. Im übrigen liegen die Verhältnisse in bezug auf die erreichten Vorteile ähnlich, wenn die erfindungsgemäße Vorrichtung nicht bei einer langgestreckten Operationswunde, sondern bei einer Defektwunde eingesetzt wird.

Im einzelnen bestehen im Rahmen der Erfindung mehrere Möglichkeiten der weiteren Ausbildung und Ausgestaltung der Vorrichtung insgesamt und der einzelnen Bauteile. In bezug auf die Verschlußgliederreihen ist eine bevorzugte Ausführungsform der Erfindung dadurch gekennzeichnet, daß die Verschlußgliederreihen als kontinuierliche Verschlußgliederreihen aus Polyamid oder Polyester, ausgeführt sind, und zwar aus Monofilament bestehen. Das Kunststoffmonofilament soll eine Fadendicke von etwa 0,5 mm oder weniger aufweisen. Die Verbindung der Verschlußgliederreihen mit den Tragbändern ist im Rahmen der Erfindung weitgehend beliebig. Eine bevorzugte Ausführungsform der Erfindung, die die schon beschriebenen Vorteile in besondere Weise fördert, ist dadurch gekennzeichnet, daß die Verschlußgliederreihen und die Tragbänder durch Weben vereinigt sind. Eine solche Verbindung der Verschlußgliederreihen mit den Tragbändern führt zu mechanischen Parametern des Reißverschlusses und der Vorrichtung insgesamt, die die beschriebenen Effekte, insbesondere in bezug auf Stauchfestigkeit und Schienungseffekte im gekuppelten Zustand unterstützen. Die Verschlußgliederreihen besitzen im gekuppelten Zustand zweckmäßigerweise eine Breite von etwa 5 mm.

Die Tragbänder können aus den verschiedensten Materialen bestehen, die in der Textiltechnik und insbesondere in der Verbandstofftechnik üblich sind. Nach bevorzugter Ausführungsform der Erfindung bestehen die Tragbänder aus Polyester oder Polyamid.

Bei der erfindungsgemäßen Vorrichtung sollen die wundfreiraumbildenden Distanzstreifen von der Mittellinie der gekuppelten Verschlußgliederreihe im allgemeinen einen Abstand von 4 bis 10 mm, vorzugsweise einen Abstand von etwa 7 mm, aufweisen. Die wundfreiraumbildenden Distanzstreifen besitzen zweckmäßigerweise eine Dicke von 0,5 bis 1,5 mm, vorzugsweise von etwa 1 mm. Eine bevorzugte Ausführungsform der Erfindung ist fernerhin dadurch gekennzeichnet, daß die wundfreiraumbildenden Distanzsteifen hautseitig klebend ausgebildet sind, wodurch die schon beschriebenen Verhältnisse beim Schließen des Reißverschlusses einer erfindungsgemäßen Vorrichtung und beim geschlossenen Reißverschluß verbessert werden.

Eine verschlossene Wunde sondert häufig Sekrete ab. Das stört bei der Verwendung einer erfindungsgemäßen Vorrichtung schon deschalb nicht, weil Sekrete über den Wundfreiraum abfließen. In bezug auf den Abfluß von Sekreten ist eine bevorzugte Ausführungsform der Erfindung weiterhin dadurch gekennzeichnet, daß die Tragbänder im Bereich zwischen den Verschlußgliederreihen und den wundfreiraumbildenden Distanzstreifen als Drainagebereiche ausgebildet sind, z.B. mit Perforationslöchern versehen sind. In werkstoffmäßiger Hinsicht kann in bezug auf die wundfreiraumbildenden Distanzstreifen mit den verschiedensten Werkstoffen gearbeitet werden. Auch hier empfiehlt sich jedoch solche einzusetzen, die in der Verbandstofftechnik bereits bewährt sind. In diesem Zusammenhang empfiehlt die Erfindung, daß die wundfreiraumbildenden Distanzstreifen als elastische Schaumstoffklebestreifen in Form von Mikroschaumpflaster ausgeführt sind, wie sie in der medizinischen Verbandstofftechnik übliche sind. Im allgemeinen besteht der Mikroschaum aus geschlossenen Polyvinylchloridzellen oder Polyäthylenzellen. Die Klebestreifen können zur Befestigung der Tragbänder längsdes Wundrandes ebenfalls als elastische Schaumstoffstreifen, insbesondere in Form von Mikroschaumpflastern, ausgeführt sein. Es kann auch ein einheitlicher Streifen sowohl als Distanzstreifen als auch als Klebestreifen eingesetzt sein. Tatsächlich besitzen solche Schaumstoffstreifen ein sehr hautähnliches Dehnungsverhalten. Es besteht aber auch die Möglichkeit, die Anordnung so zu treffen, daß die Klebestreifen zur Befestigung der Tragbänder längs des Wundrandes als unterseitig klebende Verbandmullstreifen ausgeführt sind, die, bezogen auf den Reißverschluß, in Längsrichtung dehnungsarm, in Querrichtung aber dehnbar sind. Es versteht sich, daß die Tragbänder mit dem Klebestreifen verbunden sein müssen. Das kann auf verschiedene Weise, z. B. durch Kleben, Schweißen, durch Nähnaht oder Heftnaht oder eine Kombination dieser Maßnahmen geschehen.

Wird die erfindungsgemäße Vorrichtung appliziert und die Wunde in der beschriebenen Weise durch Schließen des Reißverschlusses verschlossen, so werden die dabei auftretenden, in Längsrichtung des Reißverschlusses wirkenden Längszugkräfte zweckmäßigerweise nicht über die Klebesteifen von der Haut aufgenommen, sondern vielmehr abgeleitet. Dazu empfiehlt die Erfindung, daß die Tragbänder am geschlossen und/oder am offenen Ende des Reißverschlusses eine Halteschlaufe besitzen, die zur Aufnahme der Zugbeanspruchungen beim Schließen bzw. auch beim Öffnen des Reißverschlusses eingerichtet sind. Die Querzugkräfte werden jedenfalls von den Klebestreifen aufgenommen.

Die Tragbänder des Reißverschlusses der erfindungsgemäßen Vorrichtung können als ebene Bänder ausgeführt sein und in einer Ebene liegen, wobei die Verschlußgliederreihen von den Tragbändern hautseitig gleichsam unterfaßt sind. Es besteht aber auch die Möglichkeit, mit sogenannten verdeckten Reißverschlüssen zu arbeiten. Diese Ausführungsform der erfindungsgemäßen Vorrichtung ist dadurch gekennzeichnet, daß die Tragbänder im Bereich der Verschlußgliederreihen um eine Längsachse U-förmig abgebogen sind, daß die Verschlußgliederreihen auf der U-förmigen Abbiegung angeordnet und daß die U-förmigen Abbiegungen zur Dicke der Distanzstreifen beitragen oder diese bilden.

Im folgenden wird die Erfindung anhand einer lediglich ein Ausführungsbeispiel darstellenden Zeichnung ausführlicher erläutert. Es zeigen in schematischer Darstellung

Fig. 1　eine Draufsicht auf eine erfindungsgemäße Vorrichtung,

Fig. 2　in Gegenüber der Fig. 1 vergrößertem Maßstab einen Schnitt A-A durch den Gegenstand der Fig. 2,

Fig. 3　entsprechend der Fig. 2 einen Schnitt durch eine erfindungsgemäße Vorrichtung anderen Aufbaus, und

Fig. 4　das Verschließen einer Operationswunde mit Hilfe einer erfindungsgemäßen Vorrichtung.

Die in den Figuren dargestellte Vorrichtung dient zum Verschließen einer Wunde, insbesondere einer hauptsächlich in gerader Richtung verlaufender Operationswunde. Zum grundsätzlichen Aufbau gehören ein aus zwei textilen Tragbändern 1 und daran angeordneten Verschlußgliederreihen 2 sowie einem Reißverschlußschieber 3 aufgebauter Reißverschluß 1, 2, 3 und eine Einrichtung 4 zur Befestigung der Tragbänder 1 des Reißverschlusses 1, 2, 3 auf der Haut eines Patienten oder eines Tieres längs der Wundränder.

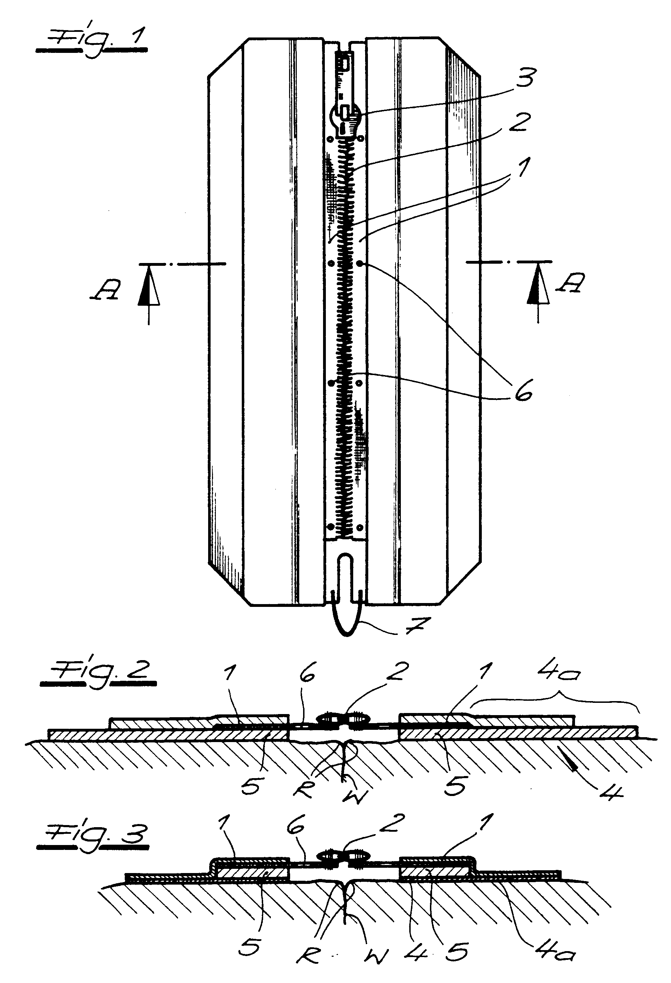

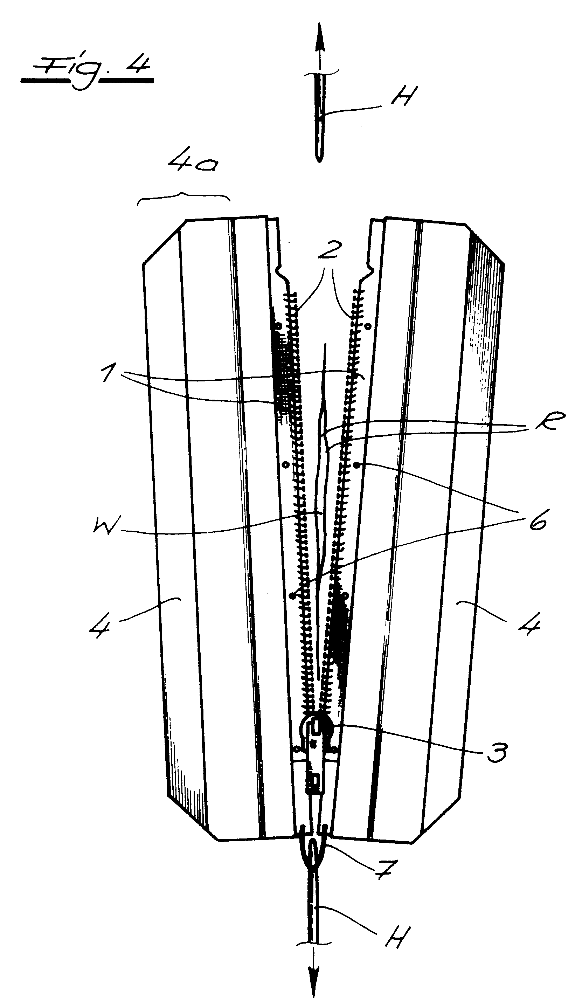

Aus einer vergleichenden Betrachtung der Fig. 1 bis 3 entnimmt man, daß der Reißverschluß 1, 2, 3 in bezug auf die Verschlußgliederreihen 2 und die Tragbänder 1 ein für textile Kleidungsstücke üblicher, flexibler Reißverschluß mit dehnungsarmen Tragbändern 1 und im gekuppelten Zustand stauchfreien Verschlußgliederreihen 2 ist. Die Fig. 2 und 3 machen deutlich, daß auf der der Wunde zugekehrten Unterseite mit Abstand von den Verschlußgliederreihen 2 auf den Tragbändern 1 wundfreiraumbildende Distanzstreifen 5 angeordnet sind. Die Einrichtung zur Befestigung der Tragbänder längs des Wundrandes besteht aus mit den Tragbändern 1 verbundenen Klebestreifen 4 mit hautseitiger hypoallergener Klebeschicht. Ein Überstand 4a steht seitlich über die Tragbänder 1 über. Die Tragbänder 1 sind von dem Klebestreifen 4 zumindest über einen Teil ihrer Breite unterlegt und die Klebestreifen 4 sind in ihrem der Haut zugewandten Bereich mit einer Breite versehen, die zur Aufnahme der Querzugkräfte beim Schließen einer Wunde und bei durch den Reißverschluß 1, 2, 3 geschlossener Wunde geeignet sind.

Im Ausführungsbeispiel und nach bevorzugter Ausführungsform der Erfindung sind die Verschlußgliederreihen 2 als kontinuierliche Verschlußgliederreihen aus Kunststoffmonofilament ausgeführt, sie bestehen beispielsweise aus Polyamid oder Polyester. Die Fadendicke des Kunststoffmonofilamentes mag etwa 0,5 mm betragen. Im übrigen sind die Verschlußgliederreihen 2 mit den Tragbändern durch Weben vereinigt. Sie besitzen im gekuppelten Zustand eine Breite von etwa 5 mm. Auch die Tragbänder 1 mögen aus Polyamid oder Polyester bestehen.

Die wundfreiraumbildenden Distanzstreifen 5 sind von der Mittellinie der gekuppelten Verschlußgliederreihen 2 entfernt angeordnet, und zwar mit einem Abstand von 4 bis 6 mm. Im Ausführungsbeispiel mag der Abstand etwa 7 mm betragen. Die wundfreiraumbildenden Distanzstreifen 5 besitzen im übrigen eine Dicke von 0,5 bis 1,5 mm. Sie mag im Ausführungsbeispiel 1 mm sein. Die Distanzstreifen 5 sind unterseitig klebend.

Insbesondere in den Fig. 1 und 4 erkennt man, daß die Tragbänder 1 im Bereich zwischen den Verschlußgliederreihen 2 und den wundfreiraumbildenden Distanzstreifen 5 als Drainagebereiche ausgebildet sind und zu diesem Zweck mit Perforationslöchern 6 versehen sind.

Bei der Ausführungsform nach den Fig. 1 und 2 sind die wundfreiraumbildenden Distanzstreifen 5 als elastische Schaumstoffklebestreifen in Form von Mikroschaumpflaster ausgeführt, wie sie in der Verbandstofftechnik üblich sind. Der Mikroschaum besteht beispielsweise aus Polyvinylchloridzellen. Die Anordnung ist im Detail so getroffen, daß die Tragbänder 1 mit einem Schaumstoffstreifen 5 unterlegt sind, der außer der hautseitigen Klebeschicht eine tragbandseitige Klebeschicht aufweist, und zwar zumindest im Tragbandbereich, und daß auf die hautabgewandte Seite dieser Schaumstoffstreifen 5 jeweils ein weiterer Schaumstoffstreifen aufgeklebt ist, der die Tragbänder 1 bis zu dem Drainagebereich mit den Performationen 6 abklebt. Die Anordnung ist in den Fig. 1 und 2 so getroffen, daß die Klebestreifen 4 zur Befestigung der Tragbänder 1 längs des Wundrandes ebenfalls als elastische Schaumstoffstreifen in Form von Mikroschaum ausgeführt sind. Der Distanzstreifen 5 geht in den Klebestreifen 4 über. Demgegenüber zeigt die Fig. 3 die Ausführungsform, bei der die Klebestreifen 4 zur Befestigung der Tragbänder 1 längs des Wundrandes als unterseitig klebende Verbandmullstreifen ausgeführt sind, die, bezogen auf den Reißverschluß 1, 2, 3, in Längsrichtung dehnungsarm, in Querrichtung aber dehnbar sind.

Im Ausführungsbeispiel sind die Tragbänder 1 und die Klebestreifen 4 durch Kleben miteinander verbunden. Sie können aber auch oder zusätzlich durch eine Nähnaht oder Heftnaht mit dem Klebestreifen vereinigt sein.

In der Fig. 1 wurde angedeutet, daß die Tragbänder am geschlossenen Ende mit einer Halteschlaufe 7 versehen sein können, die zur Aufnahme der Längszugbeanspruchungen beim Schließen des Reißverschlusses 1, 2 3, eingerichtet sind, wobei der Operateur diese Halteschlaufe 7 ergreift, wenn er andererseits zum Verschließen der Wunde den Reißverschlußschieber 3 betätigt.

In der Fig 4 erkennt man, wie zur Applikation einer Vorrichtung des beschriebenen Aufbaus bei einer hauptsächlich in Längsrichtung verlaufenden Operationswunde W mit zunächst klaffender Wundfläche verfahren wird. Die Fig. 4 zeigt, daß die Operationswunde W mittels Einzinkerhäkchen H in Längsrichtung gestreckt wurde Dadurch wurde die Wundfläche verschmälert. Längs des Wundrandes R der verschmälerten Wundfläche wurde ein Klebestreifen 4 der Vorrichtung bei geöffnetem Reißverschluß 1, 2, 3 auf die Haut aufgeklebt, und zwar so, daß der Abstand des Wundrandes R vom Rand des Distanzstreifens 5 etwa der halben Breite des Wundfreiraumes zwischen den Distanzstreifen 5 entspricht. Danach wurde längs des gegenüberliegenden Wundrandes R der verschmälerten Wundfläche der andere Klebestreifen 4 auf die Haut in entsprechender Weise aufgeklebt. Die Fig. 4 zeigt die Maßnahmen unmittelbar vor dem Verschließen der Operationswunde W durch Schieberbetätigung. Die Operationswunde W wird also verschlossen, indem der Schieber 3 in Richtung der Fig. 4 von unten nach oben bewegt wird. Man erreicht, daß die Wundränder R gegeneinander gedrückt werden. Im übrigen erkennt man in der Fig. 4, daß der Reißverschluß 1, 2, 3 der Vorrichtung um etwa 4 cm länger ist als die Wunde W und nach oben und unten um etwa 2 cm übersteht. Es versteht sich, daß die für die Befestigung der Vorrichtung auf der Haut bestimmten klebenden Flächen in üblicher Weise von einer Folie abgedeckt sind, wobei diese Folie unmittelbar vor der Applikation der Vorrichtung entfernt wird.

**Patentansprüche**

1. Vorrichtung zum Verschließen einer Wunde, insbesondere einer hauptsächlich in gerader Richtung verlaufenden Operationswunde, - mit aus zwei textilen Tragbändern und daran angeordneten Verschlußgliederreihen sowie einem Rießverschlußschieber aufbebautem Reißverschluß,
   wobei der Reißverschluß eine Einrichtung zur Klebverbindung mit der Haut eines Patienten oder eines Tieres längs des Wundrandes aufweist,
   **gekennzeichnet durch** die Kombination der folgenden Merkmale:
      1) der Reißverschluß (1, 2, 3) ist in bezug auf die Verschlußgliederreihen (2) und die Tragbänder (1) ein für textile Kleidungsstükke üblicher, flexibler Reißverschluß mit dehnungsarmen Tragbändern (1) und im gekuppelten Zustand stauchfesten Verschlußgliederreihen (2) aus Polyester oder Polyamid,
      2) auf der der Wunde zugekehrten Unterseite sind mit Abstand von den Verschlußgliederreihen (2) auf den Tragbändern wundfreiraumbildende Distanzstreifen (5) angeordnet,

3) die Einrichtung zur Klebverbindung besteht aus mit den Tragbändern (1) verbundenen, mit einem Überstand (4a) über die Tragbänder (1) überstehenden Klebestreifen (4) mit hautseitiger hypoallergener Klebeschicht,

wobei die Tragbänder (1) von den Klebestreifen (4) zumindest über einen Teil ihrer Breite unterlegt sind und die Klebestreifen (4) einschließlich der Überstände (4a) eine Breite aufweisen, die zur Aufnahme der Querzugkräfte bei durch den Reißverschluß (1, 2, 3) geschlossener Wunde mit gegeneinandergedrückten Wundrändern geeignet ist.

2. Vorrichtung nach Anspruch 1, dadurch gekennzeichnet, daß die Verschlußgliederreihen (2) als kontinuierliche Verschlußgliederreihen aus Kunststoffmonofilament ausgeführt sind, welches aus Polyamid oder Polyester besteht.

3. Vorrichtung nach Anspruch 2, dadurch gekennzeichnet, daß das Kunststoffmonofilament der Verschlußgliederreihen (2) eine Fadendicke von 0,5 mm oder weniger aufweist.

4. Verfahren nach einem der Ansprüche 1 bis 3, dadurch gekennzeichnet, daß die Verschlußgliederreihen (2) und die Tragbänder (1) durch Weben vereinigt sind.

5. Verfahren nach einem der Ansprüche 1 bis 4, dadurch gekennzeichnet, daß die Verschlußgliederreihen (2) in gekuppeltem Zustand eine Breite von etwa 5 mm aufweisen.

6. Vorrichtung nach einem der Ansprüche 1 bis 5, dadurch gekennzeichnet, daß die Tragbänder (1) aus Polyester oder Polyamid bestehen.

7. Vorrichtung nach einem der Ansprüche 1 bis 6, dadurch gekennzeichnet, daß die wundfreiraumbildenden Distanzstreifen (5) von der Mittellinie der gekuppelten Verschlußgliederreihen (2) einen Abstand von 4 bis 10 mm, vorzugsweise von etwa 7 mm aufweisen.

8. Vorrichtung nach einem der Ansprüche 1 bis 7, dadurch gekennzeichnet, daß die wundfreiraumbildenden Distanzstreifen (5) eine Dicke von 0,5 bis 1,5 mm, vorzugsweise von etwa 1mm, aufweisen.

9. Vorrichtung nach einem der Ansprüche 1 bis 8, dadurch gekennzeichnet, daß die wundfreiraumbildenden Distanzstreifen (5) hautseitig klebend ausgeführt sind.

10. Vorrichtung nach einem der Ansprüche 1 bis 9, dadurch gekennzeichnet, daß die Tragbänder (1) in Bereich zwischen den Verschlußgliederreihen und den wundfreiraumbildenden Distanzstreifen (5) als Drainagebereiche ausgebildet sind, z. B. mit Perforationslöchern (6) versehen sind.

11. Vorrichtung nach einem der Ansprüche 1 bis 10, dadurch gekennzeichnet, daß die wundfreiraumbildenden Distanzstreifen (5) als elastische Schaumstoffklebestreifen in Form von Mikroschaumpflaster ausgeführt sind, wobei die Schaumstoffklebestreifen z. B. aus geschlossenen Polyvinylchloridzellen oder Polyäthylenzellen bestehen.

12. Vorrichtung nach einem der Ansprüche 1 bis 11, dadurch gekennzeichnet, daß die Klebestreifen (4) zur Befestigung der Tragbänder längs der Wundränder ebenfalls als elastische Schaumstoffklebestreifen in Form von Mikroschaumpflaster ausgeführt sind und in die Distanzstreifen (5) übergehen.

13. Vorrichtung nach einem der Ansprüche 1 bis 11, dadurch gekennzeichnet, daß die Klebestreifen (4) zur Befestigung der Tragbänder längs der Wundrandes als unterseitig klebende Verbandmullstreifen ausgeführt sind, die, bezogen auf den Reißverschluß (1, 2, 3), in Längsrichtung dehnungsarm, in Querrichtung dehnbar sind.

14. Vorrichtung nach einem der Ansprüche 1 bis 13, dadurch gekennzeichnet, daß die Tragbänder (1) und die Klebestreifen (4) durch Schweißen und/oder Kleben miteinander verbunden sind.

15. Vorrichtung nach einem der Ansprüche 1 bis 14, dadurch gekennzeichnet, daß die Tragbänder (1) und die Klebestreifen (4) durch Nähnaht oder Heftnaht miteinander verbunden sind.

16. Vorrichtung nach einem der Ansprüche 1 bis 15, dadurch gekennzeichnet, daß die Tragbänder (1) am geschlossenen Ende und/oder am offenen Ende des Reißverschlusses (1, 2, 3) eine Halteschlaufe (7) aufweisen, an der der Reißverschluß (1, 2, 3) beim Schließen bzw. Öffnen festhaltbar ist, und die zur Aufnahme der beim Schließen und Öffnen des Reißverschlusses (1, 2, 3) auftretenden Längskräfte eingerichtet sind.

17. Vorrichtung nach einem der Ansprüche 1 bis 16, dadurch gekennzeichnet, daß die Tragbänder (1) des Reißverschlusses als ebene Bänder ausgeführt sind sowie in einer Ebene liegen und daß die Verschlußgliederreihen (2) von den Tragbändern unterfaßt sind.

18. Vorrichtung nach einem der Ansprüche 1 bis 17, dadurch gekennzeichnet, daß die Tragbänder im Bereich der Verschlußgliederreihen um eine Längsachse U-förmig abgebogen sind, daß die Verschlußgliederreihen auf der U-förmigen Abbiegung angeordnet und daß die U-förmigen Abbiegungen zur Dicke der Distanzstreifen beitragen oder diese bilden.

**Claims**

1. A device to close a wound, more specifically a substantially rectilinear surgical incision, - having
   two textile stringer tapes, rows of interlocking fastener members disposed thereon, and a sliding clasp completing the sliding clasp fastener,
   the said sliding clasp fastener having means for adhesive bonding to the skin of a patient or animal along the edges of the wound, characterised by the following combination of features:
   1) the sliding clasp fastener (1,2,3) has rows (2) of interlocking fastener members and stringer tapes (1) as in the ordinary sliding clasp fasteners used for textile garments, with low-stretch stringer tapes (1) and rows (2) of polyester or polyamide fastener members resisting compression when interlocked;
   2) spacer strips (5) forming a clearance space over the wound are provided on the underside, facing the wound clear of the rows (2) of interlocking fastener members;
   3) the means for adhesive bonding consist of adhesive strips (4) with a hypoallergenic adhesive film on the skin-facing side, attached to the stringer tapes (1) and extending laterally (at 4a) beyond the stringer tapes (1),
   the stringer tapes (1) being underlain by the adhesive strips (4) over at least a part of their breadth, and the breadth of the adhesive strips (4) including the overlaps (4a) being adapted to withstand the transverse tensile forces when the wound is closed by the sliding clasp fastener (1,2,3) and the wound edges are compressed together.

2. A device as in Claim 1, characterised in that the rows (2) of interlocking fastener members are made in continuous form from synthetic monofilaments of polyamide or polyester.

3. A device as in Claim 2, characterised in that the synthetic monofilament in the rows (2) of interlocking fastener members is 0.5 mm or less in diameter.

4. A device as in any of Claims 1 to 3, characterised in that the rows (2) of interlocking fastener members and the stringer tapes (1) are united by weaving.

5. A device as in any of Claims 1 to 4, characterised in that in the interlocked position the rows (2) of interlocking fastener members have a breadth of about 5 mm.

6. A device as in any of Claims 1 to 5, characterised in that the stringer tapes (1) are made from polyester or polyamide.

7. A device as in any of Claims 1 to 6, characterised in that the spacer strips (5), used to form the clearance space over the wound, start 4 to 10 mm off the centreline of the interlocked fastener members (2), preferably about 7 mm therefrom.

8. A device as in any of Claims 1 to 7, characterised in that the thickness of the spacer strips (5), used to form the clearance space over the wound, is between 0.5 and 1.5 mm, preferably about 1 mm.

9. A device as in any of Claims 1 to 8, characterised in that the spacer strips (5), used to form the clearance space over the wound, are adhesive on the skin-facing side.

10. A device as in any of Claims 1 to 9, characterised in that, in the zone between the rows of interlocking fastener members and the spacer strips (5) forming the clearance space, the stringer tapes (1) are adapted as drainage zones, for example provided with perforated holes (6).

11. A device as in any of Claims 1 to 10, characterised in that the spacer strips (5), forming the clearance space, are made as adhesive elastic foam plastic strips of the microfoam plaster type, e.g. consisting of cells of polyvinylchloride or polyethylene.

12. A device as in any of Claims 1 to 11, characterised in that the adhesive strips (4), used to secure the carrier strips along the wound edges, are also made from adhesive elastic foam plastics material in the form of microfoam plasters running into the spacer strips (5).

**13.** A device as in any of Claims 1 to 11, characterised in that the adhesive strips (4) used to secure the stringer tapes along the wound edges are made from surgical gauze strips with an adhesive underside, and are relatively rigid in the longitudinal direction, relative to the sliding clasp fastener (1,2,3) but capable of expanding and contracting in the transverse direction.

**14.** A device as in any of Claims 1 to 13, characterised in that the stringer tapes (1) and the adhesive strips (4) are bonded together by welding and/or adhesive bonding.

**15.** A device as in any of Claims 1 to 14, characterised in that the stringer tapes (1) and the adhesive strips (4) are bonded together by sewing or stitching.

**16.** A device as in any of Claims 1 to 15, characterised in that the stringer tapes (1) are provided at the closed and/or open ends of the sliding clasp fastener (1,2,3) with holding loops (7), adapted to immobilise the sliding clasp fastener (1,2,3) during closure and/or opening, and to withstand the longitudinal forces set up when the sliding clasp fastener (1,2,3) is closed and opened.

**17.** A device as in any of Claims 1 to 16, characterised in that the stringer tapes (1) of the sliding clasp fastener are designed as flat strips, to lie in a single plane, so that the rows (2) of interlocking members are held up by the stringer tapes (1).

**18.** A device as in any of Claims 1 to 7, characterised in that the stringer tapes in the vicinity of the rows of interlocking members are folded longitudinally back into a U-shape, the rows of interlocking fastener members being disposed on the U-shaped fold, and the U-shaped folds contributing to or constituting the thickness of the spacer strips.

**Revendications**

**1.** Dispositif pour fermer une plaie, notamment une plaie opératoire qui s'étend principalement selon une configuration rectiligne, - comportant une fermeture à glissière constituée par deux bandes textiles de support et par des séries d'éléments de fermeture disposés sur les bandes, et un curseur, la fermeture à glissière comportant un dispositif pour établir une liaison par adhérence avec la peau d'un patient ou d'un animal le long du bord de la blessure,
caractérisé par la combinaison des caractéristiques suivantes:
1) la fermeture à glissière (1,2,3) est une fermeture à glissière flexible, usuelle pour des pièces de vêtements textiles et comportant des bandes de support (1) faiblement extensibles et des séries d'éléments de fermeture (2) qui sont réalisés en polyester ou en polyamide et résistent à l'écrasement à l'état accouplé,
2) sur la face inférieure tournée vers la blessure, des bandes-entretoises (5) laissant subsister un espace dégagé au niveau de la blessure sont disposées sur les bandes de support, à distance des séries d'éléments de fermeture (2),
3) le dispositif servant à établir une liaison par adhérence est constitué par des bandes adhésives (4) qui sont réunies aux bandes de support (1), débordent sur une distance (4a) par rapport aux bandes de support (1) et comportent une couche d'adhésif hypoallergénique tournée du côté de la peau,
les bandes adhésives (4) s'étendant au-dessous des bandes de support (1) sur au moins une partie de leur largeur et possédant, y compris les distances de débordement (4a), une largeur qui est appropriée pour l'absorption des forces de traction transversales dans le cas d'une blessure fermée par la fermeture à glissière (1,2,3) et dont les bords sont repoussés l'un contre l'autre.

**2.** Dispositif selon la revendication 1, caractérisé en ce que les séries d'éléments de fermeture (2) sont réalisées sous la forme de séries continues d'éléments de fermeture formées d'un monofilament de matière plastique, qui est constitué par du polyamide ou du polyester.

**3.** Dispositif selon la revendication 2, caractérisé en ce que le monofilament de matière plastique des séries d'éléments de fermeture (2) possède une épaisseur de fil de 0,5 mm ou moins.

**4.** Procédé selon les revendications 1 à 3, caractérisé en ce que les séries d'éléments de fermeture (2) et les bandes de support (1) sont réunies par tissage.

**5.** Procédé selon l'une des revendications 1 à 4, caractérisé en ce que les séries d'éléments de fermeture possèdent, à l'état accouplé, une largeur d'environ 5 mm.

6. Dispositif selon l'une des revendications 1 à 5, caractérisé en ce que les bandes de support (1) sont réalisées en polyester ou en polyamide.

7. Dispositif selon l'une des revendications 1 à 6, caractérisé en ce que les bandes-entretoises (5), qui délimitent un espace dégagé au niveau de la blessure, sont séparées de la ligne médiane des séries d'éléments de fermeture à l'état accouplé (2) d'une distance comprise entre 4 et 10 mm égale de préférence à environ 7 mm.

8. Dispositif selon l'une des revendications 1 à 7, caractérisé en ce que les bandes-entretoises (5), qui délimitent un espace dégagé au niveau de la blessure, possèdent une épaisseur comprise entre 0,5 et 1,5 mm et égale de préférence à environ 1 mm.

9. Dispositif selon l'une des revendications 1 à 8, caractérisé en ce que les bandes-entretoises (5), qui délimitent un espace dégagé au niveau de la blessure, sont agencées de manière à être adhésives sur leur surface principale.

10. Dispositif selon l'une des revendications 1 à 9, caractérisé en ce que les bandes de support (1) sont réalisées, dans la zone située entre les séries d'éléments de fermeture et les bandes-entretoises (5), qui délimitent un espace dégagé au niveau de la blessure, sont agencées sous la forme de zones de drain, comportant par exemple des perforations (6).

11. Dispositif selon l'une des revendications 1 à 10, caractérisé en ce que les bandes-entretoises (5), qui délimitent un espace dégagé au niveau de la blessure, sont réalisées sous la forme de bandes adhésives élastiques en matériau mousse sous la forme d'un emplâtre à micromousse, les bandes adhésives en matériau mousse étant constituées par exemple par des cellules fermées de polychlorure de vinyle ou de polyéthylène.

12. Dispositif selon l'une des revendications 1 à 11, caractérisé en ce que les bandes adhésives (4) servant à fixer les bandes de support le long des bords de la blessure sont également réalisées sous la forme de bandes adhésives élastiques en matériau mousse se présentant sous la forme d'un emplâtre à micromousse et se prolongent par les bandes-entretoises (5).

13. Dispositif selon l'une des revendications 1 à 11, caractérisé en ce que les bandes adhésives (4) servant à fixer les bandes de support le long du bord de la blessure sont réalisées sous la forme de bandes de mousseline composite, dont la face inférieure est adhésive et qui sont extensibles dans la direction transversale et peu extensibles dans la direction longitudinale de la fermeture à glissière (1,2,3).

14. Dispositif selon l'une des revendications 1 à 13, caractérisé en ce que les bandes de support (1) et les bandes adhésives (4) sont réunies entre elles par soudage et/ou collage.

15. Dispositif selon l'une des revendications 1 à 14, caractérisé en ce que les bandes de support (1) et les bandes adhésives (4) sont réunies entre elles par un fil de couture ou par un fil de faufilage.

16. Dispositif selon l'une des revendications 1 à 15, caractérisé en ce que les bandes de support (1) possèdent, à l'extrémité fermée et/ou à l'extrémité ouverte de la fermeture à glissière (1,2,3), une boucle de retenue (7), à laquellle la fermeture à glissière (1,2,3) peut être fixée lors de la fermeture ou de l'ouverture, et qui est agencée de manière à absorber les forces longitudinales qui apparaissent lors de la fermeture et de l'ouverture de la fermeture à glissière (1,2,3).

17. Dispositif selon l'une des revendications 1 à 16, caractérisé en ce que les bandes de support (1) de la fermeture à glissière sont réalisées sous la forme de bandes plates et sont situées dans un plan et que les bandes de support s'engagent au-dessous des séries d'éléments de fermeture (2).

18. Dispositif selon l'une des revendications 1 à 17, caractérisé en ce que les bandes de support sont repliées en forme de U autour d'un axe longitudinal au niveau des séries d'éléments de fermeture, que les séries d'éléments de fermeture sont disposées sur la partie repliée en forme de U et que les parties repliées en forme de U contribuent à fournir l'épaisseur des bandes-entretoises ou forment ces dernières.

Fig. 1

Fig. 2

Fig. 3

Fig. 4